# EUROPÄISCHE PATENTSCHRIFT

(11) **EP 0 571 941 B1**
(45) Veröffentlichungstag und Bekanntmachung des Hinweises auf die Patenterteilung: **05.11.1997**
(21) Anmeldenummer: 93108405.7
(22) Anmeldetag: 25.05.1993
(51) Int. Cl.: G01N 33/52

(54) **Vliesschicht enthaltender Testträger**
Non-woven fabric layer containing test carrier
Support d'essai contenant une couche de tissu non-tisse

(30) Priorität: 29.05.1992 DE 4217732
(43) Veröffentlichungstag der Anmeldung: 01.12.1993
(73) Patentinhaber: BOEHRINGER MANNHEIM GMBH, 68298 Mannheim (DE)
(72) Erfinder: Lerch, Rolf, W-6804 Ilvesheim (DE); Macho, Heinz, W-6149 Fürth-Fahrenbach (DE)

(56) Entgegenhaltungen:
- EP-A- 0 045 476
- EP-A- 0 209 032
- EP-A- 0 297 390
- EP-A- 0 423 784
- EP-A- 0 443 231

## Beschreibung

Die vorliegende Erfindung betrifft einen Testträger zur Bestimmung eines Analyts in flüssigen Proben enthaltend 1) eine Vliesschicht mit einem Anteil an Polyesterfasern und 2) Reagenz zur Bestimmung eines Analyts, das in Anwesenheit des Analyts eine detektierbare Reaktion eingeht, sowie ein Verfahren zur Bestimmung eines Analyts mit Hilfe dieses Testträgers. Außerdem betrifft die Erfindung ein Vlies enthaltend Fasern aus Copolyester enthaltend Terephthalsäure, Isophthalsäure und 1,4-Butandiol sowie die Verwendung von Fasern aus Copolyester enthaltend Terephthalsäure, Isophthalsäure und 1,4-Butandiol zur Herstellung eines Testträgers oder zur Herstellung eines Vlieses.

Ein Vlies ist ein Flächengebilde von losen Fasern, die regellos nebeneinander liegen. Im Gegensatz dazu sind bei Geweben oder bei Gewirken die Fasern regelmäßig und in einer bestimmten Orientierung angeordnet. Während bei Geweben und Gewirken die Fasern so angeordnet und so miteinander in Wechselwirkung treten, daß auch bereits ohne spezielle Hilfsmittel diese Strukturen einen großen inneren Zusammenhalt aufweisen, ist es aus der textilverarbeitenden Industrie bekannt, Vliese mit Schmelzklebefasern zu verfestigen, um so stabilere textile Strukturen zu erhalten. Copolyester-Schmelzklebefasern der Firma Ems-Grilon S.A., Domat/Ems, Schweiz sind hierfür als geeignet bekannt. Eine Anwendung dieser Fasern außerhalb des textilverarbeitenden Bereiches ist nicht bekannt.

Testträger sind Analysenelemente, bei denen sich die zur Durchführung der Bestimmung eines Bestandteils flüssiger Proben notwendigen Reagenzien in oder auf festen in der Regel schichtenförmig ausgebildeten Materialien befinden. Solche Trägermaterialien können im allgemeinen saugfähige, faserige, poröse oder quellbare Materialien sein. Die Verwendung von Vliesen als schichtförmig ausgebildeten Trägermaterialien in Testträgern ist aus dem Stand der Technik bekannt.

Testträger mit Schichten aus Vliesmaterial sind beispielsweise in EP-B-0 209 032, EP-A-0 443 231 oder EP-A-0 045 476 beschrieben.

In EP-B-0 209 032 und EP-A-0 443 231 sind Testträger offenbart, in denen ein Vlies eine von mehreren Schichten darstellt. Über Zumischungen schmelzbarer Fasern zu nicht schmelzbaren Trägerfasern, die das Grundgerüst des Vlieses darstellen, ist aus diesen Schriften nichts bekannt.

EP-A-0 045 476 beschreibt den Einsatz von Glasfaservliesen in Testträgern zur Erythrozytenabtrennung aus Vollblut. Um den Zusammenhalt der Glasfasern zu verbessern, wird die Zumischung von in der Wärme verformbaren Kunststoffasern als Möglichkeit angeführt. Polyesterfasern sind allgemein genannt. Spezielle Polyesterfasern werden nicht erwähnt. Außerdem wird in dieser Anmeldung offenbart, daß Reagenzien, die die Hämolyse von Erythrozyten verhindern, die Gerinnung hemmen oder fördern oder die in der Indikatorschicht benötigt werden, mit den dortigen Reagenzien aber unverträglich sind, in oder auf dem Glasfaservlies vorliegen können.

In Testträgern zur Bestimmung von Analyten in flüssigen Proben, die Vliesmaterialien enthalten, besteht eine der Aufgaben solcher Vliese darin, Flüssigkeit und darin gelöste Stoffe gleichmäßig in dem Trägermaterial zu verteilen. Oft werden jedoch in der Flüssigkeit gelöste Substanzen nicht gleichmäßig innerhalb des Vlieses verteilt. Dies wird dadurch erklärt, daß bei Aufgabe von Flüssigkeit und darin gelösten Substanzen auf ein trockenes Vlies die Flüssigkeit sich vom Aufgabeort aus radial ausbreitet, die gelösten Substanzen je nach Affinität zum Vliesmaterial jedoch unterschiedlich schnell wandern, so daß sich innerhalb des befeuchteten Vliesmaterials Konzentrationsgradienten der in der Flüssigkeit gelösten Substanzen ergeben. Dieser Sachverhalt wird oft anschaulich als "Chromatographieeffekt" bezeichnet. Bei ungleichmäßiger Verteilung des zu bestimmenden Analyts innerhalb solcher Schichten ergibt sich dann daraus folgend oft auch, je nach dem wo und wie gemessen wird, ein unterschiedliches quantitatives Ergebnis für den zu bestimmenden Analyt.

Aufgabe der vorliegenden Erfindung war es, innerhalb von Vliesschichten Chromatographieeffekte möglichst zu vermeiden.

Diese Aufgabe wird durch die Erfindung, wie sie in den Patentansprüchen charakterisiert ist gelöst.

Gegenstand der Erfindung ist ein Testträger zur Bestimmung eines Analyts in flüssigen Proben enthaltend 1) eine Vliesschicht mit einem Anteil an Polyesterfasern und 2) Reagenz zur Bestimmung eines Analyts, das in Anwesenheit des Analyts eine detektierbare Reaktion eingeht, dadurch gekennzeichnet, daß die Polyesterfasern in der Wärme schmelzbar und Fasern aus Copolyester enthaltend Terephthalsäure, Isophthalsäure und 1,4-Butandiol sind.

Weiter ist Gegenstand der Erfindung ein Vlies enthaltend Fasern aus Copolyester enthaltend Terephthalsäure, Isophthalsäure und 1,4-Butandiol, dadurch gekennzeichnet, daß es Reagenz zur Bestimmung eines Analyts enthält, das in Anwesenheit des Analyts eine detektierbare Reaktion eingeht.

Insofern ist auch Gegenstand der Erfindung die Verwendung von Fasern aus Copolyester enthaltend Terephthalsäure, Isophthalsäure und 1,4-Butandiol zur Herstellung eines erfindungsgemäßen Testträgers bzw. eines erfindungsgemäßen Vlieses.

Schließlich ist Gegenstand der Erfindung ein Verfahren zur Bestimmung eines Analyts in flüssigen Proben, bei dem ein Testträger mit 1) einer Vliesschicht, die einen Anteil an Polyesterfasern und 2) Reagenz, das in Anwesenheit des zu bestimmenden Analyts eine detektierbare Reaktion eingeht, mit der zu untersuchenden flüssigen Probe kontaktiert und anschließend eine detektierbare Reaktion als Maß für Art und/oder Menge des Analyts gemessen wird, dadurch gekennzeichnet, daß die Vliesschicht Fasern aus Copolyester enthaltend Terephthalsäure, Isophthalsäure und 1,4-Butandiol enthält.

Überraschenderweise wurde gefunden, daß die eingangs gestellte Aufgabe gelöst werden kann, indem als Vliesmaterialien in Testträgern solche eingesetzt werden, die neben den Trägerfasern auch noch einen Anteil an in der Wärme verformbaren Polyesterfasern enthalten, wobei als in der Wärme verformbare Polyesterfasern Fasern aus Copolyester enthaltend Terephthalsäure, Isophthalsäure und 1,4-Butandiol verwendet werden. Diese Fasern sind schnellschmelzend und weisen nur eine geringe Schrumpfung in der Wärme auf (vorzugsweise weniger als 20 %). Der Schmelzbereich der Fasern aus Copolyester enthaltend Terephthalsäure , Isophthalsäure und 1,4-Butandiol reicht von etwa 100 - 220° C, vorzugsweise von ca. 140 - 200° C, ganz besonders bevorzugt von ca. 160 - 180° C je nach relativen Anteilen der Monomerkomponenten.

Der erfindungsgemäße Anteil an Fasern aus Copolyester enthaltend Terephthalsäure, Isophthalsäure und 1,4-Butandidol beträgt ca. 10 - 60 Gew.-%, vorzugsweise 30-50 Gew.-% bezogen auf das Gewicht der Gesamtfasermenge. Besonders bevorzugt für die vorliegenden Erfindung haben sich schmelzbare Copolyesterfasern eines Titers zwischen 4 und 6 dtex, ganz besonders bevorzugt zwischen 4,1 und 5,5 dtex erwiesen, wobei dieser Titer bei bekannter Dichte ein Maß für den Durchmesser von Fasern ist.

Als Trägerfasern, die das Grundgerüst des erfindungsgemäß einsetzbaren Vlieses darstellen, können grundsätzlich alle denkbaren Fasern verwendet werden, die bei Temperaturen bis 230° C noch nicht schmelzen. Erfindungsgemäß bevorzugte Fasern sind Fasern aus Glas, Polyester, Polyamid, Cellulose oder Cellulosederivaten sowie Mischungen solcher Fasern, die diese Bedingung erfüllen. Ganz besonders bevorzugt sind Trägerfasern aus schwer schmelzbarem Polyester oder Glas.

Im einfachsten Fall enthält ein schichtenförmig aufgebauter erfindungsgemäßer Testträger nur eine Schicht. Diese besteht dann aus einem Vlies mit Trägerfasern und einem Anteil an Fasern aus Copolyester enthaltend Terephthalsäure , Isophthalsäure und 1,4-Butandiol und enthält zusätzlich das zur Bestimmung eines Analyts erforderliche Reagenz, das in Anwesenheit des Analyts eine detektierbare Reaktion eingeht, wobei als detektierbare Reaktion eine solche verstanden wird, bei der in Anwesenheit des Analyts ein Signal erzeugt wird, das gemessen werden kann und das ein Maß für die Menge, beziehungsweise auch für die Art des Analyts darstellt. Üblicherweise werden kolorimetrische oder fluorimetrische Meßverfahren als Detektionsmethoden angewandt. Verfahren bei denen die Entstehung, Veränderung oder Abnahme einer Farbe beobachtet wird, sind besonders bevorzugt.

In dem Vlies des erfindungsgemäßen Testträgers kann sich das Reagenz gleichmäßig verteilt befinden, beispielsweise so wie es sich durch Imprägnierung des Vlieses mit dem Reagenz in flüssiger Form ergibt. Es kann jedoch auch auf das Vlies beschichtet sein. Außerdem kann sich das Reagenz an den Fasern des Vlieses immobilisiert befinden oder durch Flüssigkeit ablösbar aufgebracht sein.

Da Vliese als solches oft nicht ausreichend steif sind, um für die Bestimmung eines Analyts in Flüssigkeit gut gehandhabt werden zu können, kann ein erfindungsgemäßes, Reagenz enthaltendes Vlies auch auf einem steifen, an sich inerten Material befestigt sein, das die bestimmungsgemäße Handhabung des Vlieses erleichtert. Steife Kunststoffolien haben sich hierzu besonders bewährt, andere steife Materialien sind jedoch auch denkbar, beispielsweise Glas, Metall, etc.

Grundsätzlich kann das erfindungsgemäße Vlies in allen Testträgern an Stelle von bisher üblichen Vliesen eingesetzt werden. Beispielsweise ist das erfindungsgemäße Vlies in Testträgern gemäß EP-A-0 045 476, EP-B-0 209 032 oder gemäß EP-A- 0 443 231 einsetzbar. In solchen Testträgern muß das Vlies kein Reagenz zur Analytbestimmung enthalten, da es dort anstatt als Indikatorschicht auch andere Funktionen erfüllen kann, wie beispielsweise die Erythrozytenabtrennung aus Vollblut, Transport von Probenflüssigkeit zwischen zwei anderen Schichten oder Zonen eines Testträgers oder einfach auch als Saugschicht zur Entfernung überschüssiger Flüssigkeit aus einem bestimmten Bereich eines Testträgers. Testträger mit mehreren Schichten, in denen sich das Reagenz nicht in oder auf dem findungsgemäßen Vlies befindet, sondern auf einer weiteren Schicht, die ein Vlies, aber auch ein anderes schichtenförmiges Material sein kann, sollen ebenfalls vom Gegenstand der vorliegenden Testträgeransprüche umfaßt sein.

Flüssige Proben, die mit einem erfindungsgemäßen Testträger untersucht werden können, sind von ihrer Art her grundsätzlich nicht beschränkt. Es versteht sich jedoch von selbst, daß das Vliesmaterial gegenüber der zu untersuchenden Probe inert sein sollte. Üblicherweise sind Testträger für die Untersuchung von Körperflüssigkeiten, wie beispielsweise Blut, Serum, Plasma, Urin, Speichel etc. bestimmt. Auch der erfindungsgemäße Testträger ist für die Untersuchung von Blut, Plasma, Serum und Urinproben besonders geeignet. Jedoch ist auch die Verwendung zur Untersuchung anderer Flüssigkeiten möglich.

Zur Durchführung des Bestimmungsverfahrens wird der erfindungsgemäße Testträger mit der zu untersuchenden flüssigen Probe kontaktiert, beispielsweise durch Eintauchen in die zu untersuchende Flüssigkeit oder Aufgeben der zu untersuchenden Flüssigkeit auf den Testträger und anschließend eine detektierbare Reaktion als Maß für Art und/oder Menge des Analyts gemessen.

Das erfindungsgemäße Vlies zeichnet sich dadurch aus, daß praktisch kein Chromatographieeffekt auftritt. Außerdem ist die Geschwindigkeit, mit der sich Flüssigkeit in dem erfindungsgemäßen Vlies ausbreitet deutlich größer als in solchen Vliesen, die bisher übliche Polyesterfasern enthielten. Diese Eigenschaften führen dazu, daß Bestimmungsverfahren mit Hilfe des erfindungsgemäßen Vlieses schneller und genauer durchführbar sind.

### Beispiel 1

Es werden vier Vliese (a-d) hergestellt. Als Ausgangsmaterialien dienen hierzu:
a) 30 Teile Polyesterfasern 1,7/6 (Du Pont, Bad Homburg, Deutschland)
   20 Teile Viskosefasern, 1,7/6 (Rohtex Textil, Mönchengladbach, Deutschland)
   30 Teile Grilene-Fasern (Ems-Grilon S.A., Domat/Ems, Schweiz)
   20 Teile Kuralon (Polyvinylalkohol), (Rohtex Textil, Mönchengladbach, Deutschland)
b) 60 Teile Polyesterfasern 1,7/6 (Du Pont, Bad Homburg, Deutschland)
   20 Teile Viskosefasern, 1,7/6 (Rohtex Textil, Mönchengladbach, Deutschland)
   20 Teile Kuralon (Polyvinylalkohol), (Rohtex Textil, Mönchengladbach, Deutschland)
c) 50 Teile Glasfasern, Typ 108, (John Mansfield, Denver, Colorado, USA)
   50 Teile Grilenefasern, 1,7/6 (Ems-Grilon S.A., Domat/Ems, Schweiz)
   10 Teile Kuralon (Polyvinylalkohol), (Rohtex Textil, Mönchengladbach, Deutschland)
d) 100 Teile Glasfasern Typ 108, (John Mansfield, Denver, Colorado, USA)
   10 Teile Kuralon (Polyvinylalkohol), (Rohtex Textil, Mönchengladbach, Deutschland)

Die angegebenen Teile sind Gewichtsteile.

Die für die Vliese a) und c) verwendeten Grilene-Fasern sind Fasern aus Copolyester enthaltend Terephthalsäure, Isophthalsäure und 1,4-Butandiol, die hier ihren erfindungsgemäßen Einsatz finden.

Als Papiermaschine wird eine Schrägsiebmaschine (Voith, Heidenheim, Deutschland) verwendet. Die in Wasser suspendierten Fasern werden auf ein Schrägsieb gepumpt. Während die Flüssigkeit abfließt bzw. durch Vakuum abgesaugt wird, orientieren sich die Fasern auf der Sieboberfläche und werden als Vlies über Trockenzylinder getrocknet. Die Trocknung findet bei 125°C statt, bis eine Endfeuchte von 0,5 bis 1,5 Gew.-% erreicht ist. Die Absaugung und Produktionsgeschwindigkeit werden mit 2 ml pro Minute so gewählt, daß ein Material eines Flächengewichtes von 60 g/m² entsteht.

### Beispiel 2

Die in Beispiel 1 hergestellten Vliese a) bis d) wurden in 15 mm breite und 200 mm lange Streifen geschnitten. An einem Ende der Vliesstreifen wurden 50 µl einer konzentrierten Patentblaulösung aufpipetiert und anschließend bei 60° C getrocknet.
Nach dem Trocknen wurden die Streifen mit dem imprägnierten Ende 5 mm in ein Wasserbad gehängt und nach 30 mm Saughöhe die Vliese aus dem Wasser entfernt. Visuell wurde das Auswasch- und Laufverhalten der Farbe ermittelt.

Während die Vliesmuster ohne Grilene (Vliese b und d) eine deutliche Farbfront aufweisen und die Farbe an der Fließfront angereichert wird, sind an den Vliesmustern mit Zusatz von Grilene (Vliese a und c) diese Effekte deutlich weniger ausgeprägt. Die Vliese mit Grilenefasern sind dagegen über die gesamte Lauffläche homogen gefärbt. Farbstoffanreicherungen an der Fließfront sind nicht erkennbar.

## Patentansprüche

1. Testträger zur Bestimmung eines Analyts in flüssigen Proben enthaltend 1) eine Vliesschicht mit einem Anteil an Polyesterfasern und 2) Reagenz zur Bestimmung eines Analyts, das in Anwesenheit des Analyts eine detektierbare Reaktion eingeht, dadurch gekennzeichnet, daß die Polyesterfasern bei etwa 100 bis 220 ° C schmelzbar und Fasern aus Copolyester enthaltend Terephthalsäure, Isophthalsäure und 1,4-Butandiol sind.

2. Testträger gemäß Anspruch 1, dadurch gekennzeichnet, daß der Anteil an Fasern aus Copolyester enthaltend Terephthalsäure, Isophthalsäure und 1,4-Butandiol am Vliesmaterial der Schicht 10-60 Gew.-% beträgt.

3. Testträger gemäß Anspruch 1 oder 2, dadurch gekennzeichnet, daß das Vliesmaterial, neben schmelzbaren Copolyesterfasern, Glasfasern, Polyesterfasern, Polyamidfasern, Cellulosefasern oder Cellulosederivatefasern allein oder Mischungen dieser Fasern, die bis 230 ° C nicht schmelzen, enthält.

4. Vlies enthaltend Fasern aus Copolyester, enthaltend Terephthalsäure, Isophthalsäure und 1,4-Butandiol, dadurch gekennzeichnet, daß es Reagenz zur Bestimmung eines Analyts enthält, das in Anwesenheit des Analyts eine detektierbare Reaktion eingeht und die Copolyesterfasern im Bereich von etwa 100-220° C schmelzen.

5. Verwendung von Fasern aus Copolyester enthaltend Terephthalsäure, Isophthalsäure und 1,4-Butandiol zur Herstellung eines Testträgers gemäß einem der Ansprüche 1-3 oder zur Herstellung eines Vlieses gemäß Anspruch 4.

6. Verfahren zur Bestimmung eines Analyts in flüssigen Proben, bei dem ein Testträger mit 1) einer Vliesschicht, die einen Anteil an Polyesterfasern enthält und 2) Reagenz, das in Anwesenheit des zu bestimmenden Analyts eine detektierbare Reaktion eingeht, mit der zu untersuchenden flüssigen Probe kontaktiert und anschließend eine detektierbare Reaktion als Maß für Art und / oder Menge des Analyts gemessen wird, dadurch gekennzeichnet, daß die Vliesschicht Fasern aus Copolyester enthaltend Terephthalsäure, Isophthalsäure und 1,4-Butandiol enthält, die im Bereich von etwa 100-220° C schmelzen.

## Claims

1. Test carrier for the determination of an analyte in liquid samples containing 1) a fleece layer with a proportion of polyester fibres and 2) a reagent for the determination of an analyte which enters into a detectable reaction in the presence of the analyte, wherein the polyester fibres are meltable at about 100 to 220°C and are fibres made of copolyester containing terephthalic acid, isophthalic acid and 1,4-butanediol.

2. Test carrier as claimed in claim 1, wherein the proportion of fibres made of copolyester containing terephthalic acid, isophthalic acid and 1,4-butanediol in the fleece material of the layer is 10 - 60 % by weight.

3. Test carrier as claimed in claim 1 or 2, wherein the fleece material contains glass fibres, polyester fibres, polyamide fibres, cellulose fibres or cellulose derivative fibres alone or as mixtures of these fibres which do not melt up to 230°C in addition to meltable copolyester fibres.

4. Fleece which contains fibres made of copolyester containing terephthalic acid, isophthalic acid and 1,4-butanediol, wherein it contains a reagent for the determination of an analyte which enters into a detectable reaction in the presence of the analyte and the copolyester fibres melt in a range of about 100 - 220°C.

5. Use of fibres made of copolyester containing terephthalic acid, isophthalic acid and 1,4-butanediol for the production of a test carrier as claimed in one of the claims 1 - 3 or for the production of a fleece as claimed in claim 4.

6. Method for the determination of an analyte in liquid samples in which a test carrier with 1) a fleece layer which contains a proportion of polyester fibres and 2) a reagent which enters into a detectable reaction in the presence of the analyte to be determined is brought into contact with the liquid sample to be examined and subsequently a detectable reaction is measured as a measure of the type and/or amount of the analyte, wherein the fleece layer contains fibres made of copolyester containing terephthalic acid, isophthalic acid and 1,4-butanediol which melt in a range of about 100 - 220°C.

## Revendications

1. Support de test pour déterminer un analyte dans un prélèvement liquide, contenant (1) une couche de matériau non tissé ayant une portion de fibres de polyester et (2) un réactif en vue de déterminer un analyte, qui en présence de l'analyte forme une réaction détectable, caractérisé en ce que les fibres de polyester sont fusibles à une température d'environ 100°C à 200 °Cet sont un copolyester contenant de l'acide téréphtalique, de l'acide isophtalique et du 1,4-butanediol.

2. Support de test selon la revendication 1, caractérisé en ce que la portion de fibres constituées de copolyester contenant de l'acide téréphtalique, de l'acide isophtalique et du 1,4-butanediol dans le matériau non tissé de la couche est dans la gamme de 10% à 60% en poids.

3. Support de test selon la revendication 1 ou 2, caractérisé en ce que le matériau non tissé contient, outre les fibres de copolyester ffusibles des fibres de verre, des fibres de polyester, des fibres de polyamide, des fibres de cellulose ou des fibres de dérivés cellulosiques seules ou des mélanges de ces fibres, qui ne fondent pas jusqu'à 230°C.

4. Matériau non tissé contenant des fibres de copolyester, contenant de l'acide téréphtalique, de l'acide isophtalique et du 1,4-butanediol, caractérisé en ce qu'il contient un réactif pour déterminer un analyte, qui en présence de l'analyte forme une réaction détectable, et que les fibres de copolyester fondent dans la plage d'environ 100°C à 220°C.

5. Utilisation de fibres de copolyester contenant de l'acide téréphtalique, de l'acide isophtalique et du 1,4-butanediol pour la fabrication d'un support de test selon l'une quelconque des revendications 1 à 3 ou pour la fabrication d'un matériau non tissé selon la revendication 4.

6. Procédé pour déterminer un analyte dans un prélèvement liquide, dans lequel un support de test ayant (1) une couche de matériau non tissé, qui contient une portion de fibres de copolyester et (2) un réactif, qui en présence de l'analyte à déterminer forme une réaction détectable, vient en contact avec le prélèvement liquide à analyser et ensuite une réaction détectable est détectée en tant que détermination qualitative et/ou quantitative de l'analyte, caractérisé en ce que la couche de matériau non tissé contient des fibres de copolyester contenant de l'acide téréphtalique, de l'acide isophtalique et du 1,4-butanediol, qui fondent dans la plage d'environ 100 à 220°C.
